# EUROPEAN PATENT SPECIFICATION

(11) **EP 0 563 797 B1**
(45) Date of publication and mention of the grant of the patent: **05.07.2000**
(21) Application number: 93104976.1
(22) Date of filing: 25.03.1993
(51) Int. Cl.: C07D 471/04, G01N 33/68, G01N 33/72

(54) **Pyridinium derivatives**
Pyridiniumderivate
Dérivés de pyridinium

(30) Priority: 25.03.1992 JP 10039492; 24.06.1992 JP 19164992
(43) Date of publication of application: 06.10.1993
(73) Proprietor: NIPPON ZOKI PHARMACEUTICAL CO., LTD., Chuo-ku, Osaka (JP)
(72) Inventor: Nakamura, Ko, c/o Institute of Bio-Active Science, Yashiro-cho, Katoh-gun, Hyogo (JP); Hochi, Toshikazu, c/o Inst. of Bio-Active Science, Yashiro-cho, Katoh-gun, Hyogo (JP); Ienaga, Kazuharu, c/o Inst. of Bio-Active Science, Yashiro-cho, Katoh-gun, Hyogo (JP)
(74) Representative: Hansen, Bernd, Dr. Dipl.-Chem.

(56) References cited:
- EP-A- 0 288 256
- EP-A- 0 360 258
- WO-A-89/04491
- WO-A-91/02978
- CARBOHYDRATE RESEARCH vol. 182, no. 2, 1988, AMSTERDAM pages 301 - 306 BERNHARD HUBER ET AL. 'Formation of 2-(2-furoyl)-4(5)-(2-(furyl)-1H-imidazole in the Maillard reaction'

## Description

The present invention relates to pyridinium derivatives, an antibody prepared from such a derivative as a hapten, their use for diagnosis of diabetes, diabetic complications, aging, diseases accompanied by aging etc., and their use for the evaluation of effects of pharmaceuticals which are effective against such diabetes-related diseases, aging and diseases accompanied by same.

In 1968, glycosylhemoglobin (HbAlc) which is one of the minor components of hemoglobin was identified in vivo and was found to increase in patients suffering from diabetes. With this as a momentum, the biological meaning of the Maillard reaction and, particularly, the relation between aging and diabetes have been receiving public attention.

Maillard reaction may be classified into a first stage wherein a Schiff base is formed from an amino group of a protein and an aldehyde group of a reduced sugar and is stabilized as a result of an Amadori rearrangement and a second stage wherein the above is transferred, after a long series of reaction, to second stage products of the Maillard reaction which are characteristic in fluorescence, color change to brown and molecular crosslinking. The fluorescence which is known as one of the characteristic changes in the second stage products of the Maillard reaction is significantly higher in diabetic patients than in healthy and normal persons and is suggested to have a correlation with the onset of diabetic complications such as diabetic nephrosis, arteriosclerosis, nervous disturbance, retinal diseases, cataract, etc.

However, basic structures for these second-stage products of the Maillard reaction have not been clarified yet. Accordingly, there have been many ambiguous points with respect to mechanisms for the nonenzymatic saccharization and crosslinking of proteins caused by aging and diabetes.

The present inventors have conducted continued studies on the mechanisms of nonenzymatic saccharization and the crosslinking of proteins and, as a result thereof, found novel pyridinium derivatives which relate to the basic structures of the second-stage products of the Maillard reaction whereby the present invention has been accomplished.

An object of the present invention is to offer pyridinium derivatives, an antibody prepared from such a derivative as a hapten, their use for the diagnosis of diabetes, diabetic complications, aging, diseases accompanied by aging, etc., and their use for the evaluation of the effects of pharmaceuticals for diabetes-related diseases, aging, diseases accompanied by same, etc.

Accordingly, the present invention provides a pyridinium derivative represented by the general formula (I) or (II) 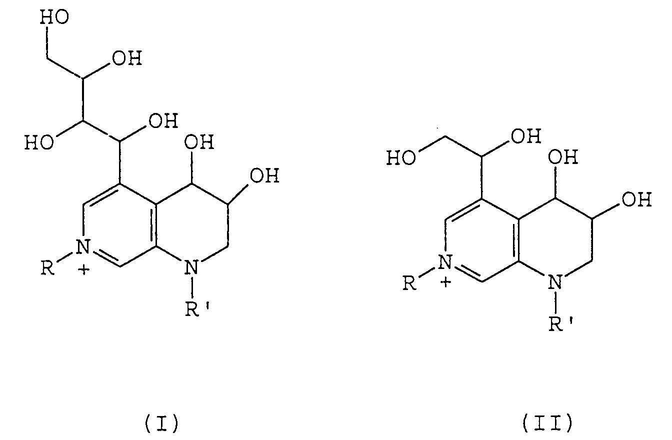 wherein R and R', which may be same or different, are linear, branched or cyclic C₁₋₆ alkyl groups optionally substituted by an amino group, a protected amino group and/or a carboxyl group, or a pharmaceutically acceptable salt thereof.

With regard to the protective groups for an amino group, those which are commonly used in the field of peptide synthesis, etc. may be utilized. They are, for example, acetyl, benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenylazobenzyloxycarbonyl, tert-butoxycarbonyl, tert-amyloxycarbonyl, p-biphenylisopropyloxycarbonyl, diisopropylmethyloxycarbonyl and formyl.

The pyridinium derivatives of the present invention include salts of the compounds represented by general formulae (I) or (II). Such salts are, for example, salts with alkali metals such as sodium, potassium and lithium; alkaline earth metals such as magnesium, calcium and barium; and other metals such as aluminum. Further examples are addition salts with acids such as hydrochloric acid, sulfuric acid, nitric acid, phosphoric acid, formic acid, acetic acid, citric acid, lactic acid, hydrobromic acid and trifluoroacetic acid; and salts with ammonia or with organic bases such as organic amines. Those salts can be prepared by conventional methods from pyridinium derivatives of the present invention in free form and vice versa.

When stereoisomers such as cis-trans isomers, optical isomers and conformational isomers exist in the compounds of the present invention, the present invention includes any of these isomers.

An example for manufacturing methods for the compounds of the present invention are given below.

A compound of the formula R-NH₂ or R'-NH₂ (wherein R and R' are the same groups as mentioned hereinabove) is made copresent with a hexose such as glucose, fructose, galactose or mannose, or an oligosaccharide consisting of at least one hexose, providing the compound of the present invention. There is no particular restriction for the reaction conditions such as reaction temperature, reaction time, pH, etc. but they may be properly established. For example, the reaction can be accelerated by heating.

The compounds of the present invention prepared as such were purified by common means such as distillation, chromatography, recrystallization, etc. and identified by means of NMR, mass analysis, fluorescence spectrum, etc.

It is possible to combine the compounds of the present invention with a carrier protein such as bovine serum albumin, keyhole limpet hemocyanin (KLH) and the like, using water-soluble carbodiimide in accordance with a usual coupling reaction to prepare an antibody in a manner known as such. Said antibody is capable of recognizing the compound of the present invention which was used for its preparation in vitro and is also capable of immunochemically discriminating the saccharized protein prepared in vitro and the crosslinked protein in the basement membrane of the glomerulus of model rats suffering from diabetic nephrosis induced by streptozotocin.

Among the compounds of the present invention represented by the general formulae (I) or (II), pyridinium derivatives wherein R and R' are alkyl having amino and/or carboxyl groups can be easily combined with a carrier protein etc. as haptens and are useful particularly for the preparation of antibodies. As to a carrier for combining with a hapten for the preparation of antibodies, the commonly-used one such as a protein (e.g. serum albumin, KLH) and polymers (e.g. polylysine) may be used. Among compounds of the present invention, those compounds which are structurally closely related to the second-stage products of the Maillard reaction in terms of chemical structures are practically preferred due to their possible use which is described in the following.

Accordingly, it is possible to conduct diagnosis of diabetes, diabetic complications (e.g. diabetic nephrosis, diabetic arteriosclerosis, diabetic nervous disturbance, diabetic cataract, diabetic retinal diseases, diabetic minute blood vessel disturbance, etc.), aging and diseases accompanied by same using the compounds of the present invention as an indicator. Moreover, it is possible to conduct an evaluation of pharmaceutical effects in the test systems in vitro and/or in vivo using the compounds of the present invention as an indicator. In addition, the antibody prepared from the compound of the present invention as a hapten can be utilized immunochemically and immunohistochemically in the above-mentioned diagnosis and evaluation and are very highly useful. The present invention is illustrated in more detail by means of the following examples.

### Example 1

Pentylamine (25 ml) and 38.7 g glucose were dissolved in 1 liter of 250 mM phosphate buffer (pH: 7.4) and allowed to stand at 37°C for 3 weeks.

The reaction solution was washed with 500 ml of ethyl acetate twice and the resulting aqueous layer was added to a column of Amberlite XAD-2 followed by thorough washing with water. The fraction eluted by methanol containing 10% of acetic acid was concentrated, the concentrate was dissolved in water and the solution was washed with ethyl acetate again. This was acidified with concentrated hydrochloric acid, extracted with n-butanol and the extract was added to a column of Amberlite XAD-2. Acetic acid (20%) was passed through the column and the fractions containing fluorescent substances were combined and concentrated. The concentrate was added to a column of reversed phase type, washed and eluted with a mixture of methanol and trifluoroacetic acid to give 1.8 g of oil. The oil was purified by separation by means of high performance liquid chromatography to give 450 mg of compound 1; 350 mg of compound 2; 50 mg of compound 3; and 30 mg of compound 4 separately. Each of them was converted to its hydrochloride.

### Example 2

alpha-Acetyllysine hydrochloride (44.9 g) was reacted with 36 g of glucose by allowing to stand at 37°C for 6 weeks in accordance with the method of Example 1. The reaction solution was not concentrated but just added to an ion exchange resin of a sulfonate type, well washed with water and eluted with 2N aqueous ammonia. The fraction was concentrated, separated/purified using the same resin (wherein a mixture of methanol and trifluoroacetic acid was used) and converted to hydrochlorides to afford 37 mg of pale yellow and sirupy compound 5; 26 mg of compound 6; and several mg of the compounds 7 and 8.

The same procedures were conducted as above using gamma-aminobutyric acid and β-alanine to give the compounds 9 to 17.

### Example 3

Instead of glucose, fructose was mixed with pentylamine and the same procedures were conducted as above. As a result of this reaction, the compounds 1 to 4 were obtained similarly to Example 1.

The physical properties of the resulting compounds in accordance with the present invention are as follows:
Compound 1:
   (3R,4S)-3,4-Dihydroxy-5-{1-[(1S,2S,3R)-1,2,3,4-tetrahydroxybutyl]}-1,7-dipentyl-1,2,3,4-tetrahydro-[1,7]naphthylidium
   Fluorescence spectrum: Em = 380 nm, Ex = 463 nm
   Mass analysis (SIMS, Glycerol): M⁺ (m/z) = 427.
Compound 2:
   (3R,4S)-3,4-Dihydroxy-5-{1-[(1R,2S,3R)-1,2,3,4-tetrahydroxybutyl]}-1,7-dipentyl-1,2,3,4-tetrahydro-[1,7]naphthylidium
   Fluorescence spectrum: Em = 379 nm, Ex = 464 nm
   Mass analysis (SIMS, Glycerol): M⁺ (m/z) = 427.
Compound 3:
   (3R,4S)-3,4-Dihydroxy-5-{1-[(1S)-1,2-dihydroxyethyl]}-1,7-dipentyl-1,2,3,4-tetrahydro-[1,7]-naphthylidium
   Fluorescence spectrum: Em = 380 nm, Ex = 464 nm
   Mass analysis (SIMS, Glycerol): M⁺ (m/z) = 367.
Compound 4:
   (3R,4S)-3,4-Dihydroxy-5-{1-[(1R)-1,2,dihydroxyethyl]}-1,7-dipentyl-1,2,3,4-tetrahydro-[1,7]-naphthylidium
   Fluorescence spectrum: Em = 380 nm, Ex = 462 nm
   Mass analysis (SIMS, Glycerol): M⁺ (m/z) = 367.
Compound 5:
   (3R,4S)-3,4-Dihydroxy-5-{1-[(1S,2S,3R-1,2,3,4-tetrahydroxybutyl]}-1,7-di[6-(N-acetyl-L-norleucyl)]-1,2,3,4-tetrahydro-[1,7]naphthylidium
   Fluorescence spectrum: Em = 379 nm, Ex = 461 nm
   Mass analysis (SMS, Glycerol): M⁺ (m/z) = 629.
Compound 6:
   (3R,4S)-3,4-Dihydroxy-5-{1-[(1R,2S,3R)-1,2,3,4-tetrahydroxybutyl]}-1,7-di[6-(N-acetyl-L-norleucyl)]-1,2,3,4-tetrahydro-[1,7]naphthylidium
   Fluorescence spectrum: Em = 377 nm, Ex = 461 nm
   Mass analysis (SIMS, Glycerol): M⁺ (m/z) = 629.
Compound 7:
   (3R,4S)-3,4-Dihydroxy-5-{1-[(1S)-1,2-dihydroxyethyl]}-1,7-di[6-(N-acetyl-L-norleucyl)]-1,2,3,4-tetrahydro-[1,7]naphthylidium
   Fluorescence spectrum: Em = 379 nm, Ex = 461 nm
   Mass analysis (SIMS, Glycerol): M⁺ (m/z) = 569.
Compound 8:
   (3R,4S)-3,4-Dihydroxy-5-{1-[(1R)-1,2-dihydroxyethyl]}-1,7-di[6-(N-acetyl-L-norleucyl)]-1,2,3,4-tetrahydro-[1,7]naphthylidium
   Fluorescence spectrum: Em = 378 nm, Ex = 462 nm
   Mass analysis (SIMS, Glycerol): M⁺ (m/z) = 569.
Compound 9:
   (3R,4S)-3,4-Dihydroxy-5-{1-[(1S,2S,3R)-1,2,3,4-tetrahydroxybutyl]}-1,7-di(3-carboxypropyl)-1,2,3,4-tetrahydro-[1,7]naphthylidium
   Fluorescence spectrum: Em = 380 nm, Ex = 464 nm
   Mass analysis (SIMS, Glycerol): M⁺ (m/z) = 443.
Compound 10:
   (3R,4S)-3,4-Dihydroxy-5-{1-[(1R,2S,3R)-1,2,3,4-tetrahydroxybutyl]}-1,7-di(3-carboxypropyl)-1,2,3,4-tetrahydro-[1,7]naphthylidium
   Fluorescence spectrum: Em = 379 nm, Ex = 463 nm
   Mass analysis (SIMS, Glycerol): M⁺ (m/z) = 443.
Compound 11:
   (3R,4S)-3,4-Dihydroxy-5-{1-[(1S)-1,2-dihydroxyethyl]}-1,7-di(3-carboxypropyl)-1,2,3,4-tetrahydro-[1,7]naphthylidium
   Fluorescence spectrum: Em = 380 nm, Ex = 462 nm
   Mass analysis (SIMS, Glycerol): M⁺ (m/z) = 383.
Compound 12:
   (3R,4S)-3,4-Dihydroxy-5-{1-[(1R)-1,2-dihydroxyethyl]}-1,7-di(3-carboxypropyl)-1,2,3,4-tetrahydro-[1,7]naphthylidium
   Fluorescence spectrum: Em = 380 nm, Ex = 463 nm
   Mass analysis (SIMS, Glycerol): M⁺ (m/z) = 383.
Compound 13:
   (3R,4S)-3,4-Dihydroxy-5-{1-[(1S,2S,3R)-1,2,3,4-tetrahydroxybutyl]}-1,7-di(2-carboxyethyl)-1,2,3,4-tetrahydro-[1,7]naphthylidium
   Fluorescence spectrum: Em = 379 nm, Ex = 463 nm
   Mass analysis (SIMS, Glycerol): M⁺ (m/z) = 429.
Compound 14:
   (3R,4S)-3,4-Dihydroxy-5-{1-[(1R,2S,3R)-1,2,3,4-tetrahydroxybutyl]}-1,7-di(2-carboxyethyl)-1,2,3,4-tetrahydro-[1,7]naphthylidium
   Fluorescence spectrum: Em = 379 nm, Ex = 464 nm
   Mass analysis (SIMS, Glycerol): M⁺ (m/z) = 429.
Compound 15:
   (3R,4S)-3,4-Dihydroxy-5-{1-[(1S)-1,2-dihydroxyethyl]}-1,7-di(2-carboxyethyl)-1,2,3,4-tetrahydro[1,7]naphthylidium
   Fluorescence spectrum: Em = 380 nm, Ex = 463 nm
   Mass analysis (SIMS, Glycerol): M⁺ (m/z) = 369.
Compound 16:
   (3R,4S)-3,4-Dihydroxy-5-{1-[(1R)-1,2-dihydroxyethyl]}-1,7-di(2-carboxyethyl)-1,2,3,4-tetrahydro-[1,7]naphthylidium
   Fluorescence spectrum: Em = 378 nm, Ex = 462 nm
   Mass analysis (SIMS, Glycerol): M⁺ (m/z) = 369.
Compound 17:
   Epimer of the compound 9 with respect to carbon atom 3.

Tables 1 to 3 show some examples of analytic results of ¹H-NMR (400 MHz, CD₃OD) and ¹³C-NMR (100 MHz, CD₃OD) spectra. TMS was used as an internal standard and the assignments in ¹³C-NMR were based upon the results of measurements of "C-H COSY" and "COLOC". Values in parentheses are coupling coefficients. Incidentally, the assignments marked with !, !!, * and ** may be replaced by each other.

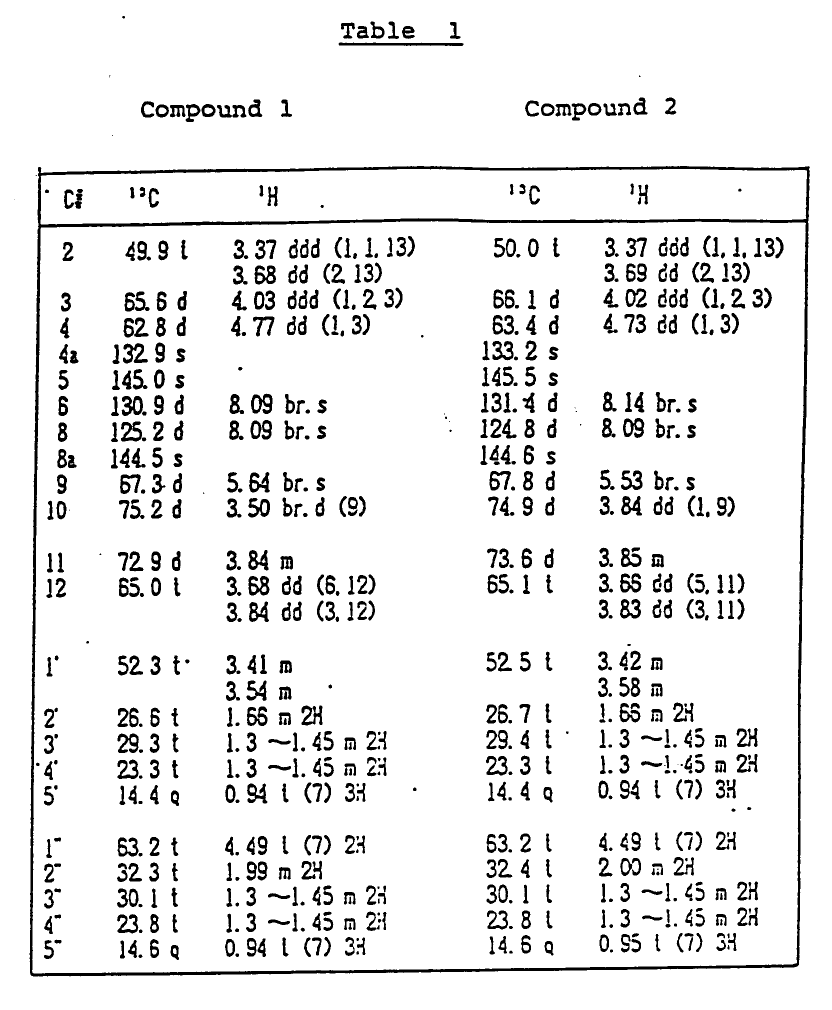

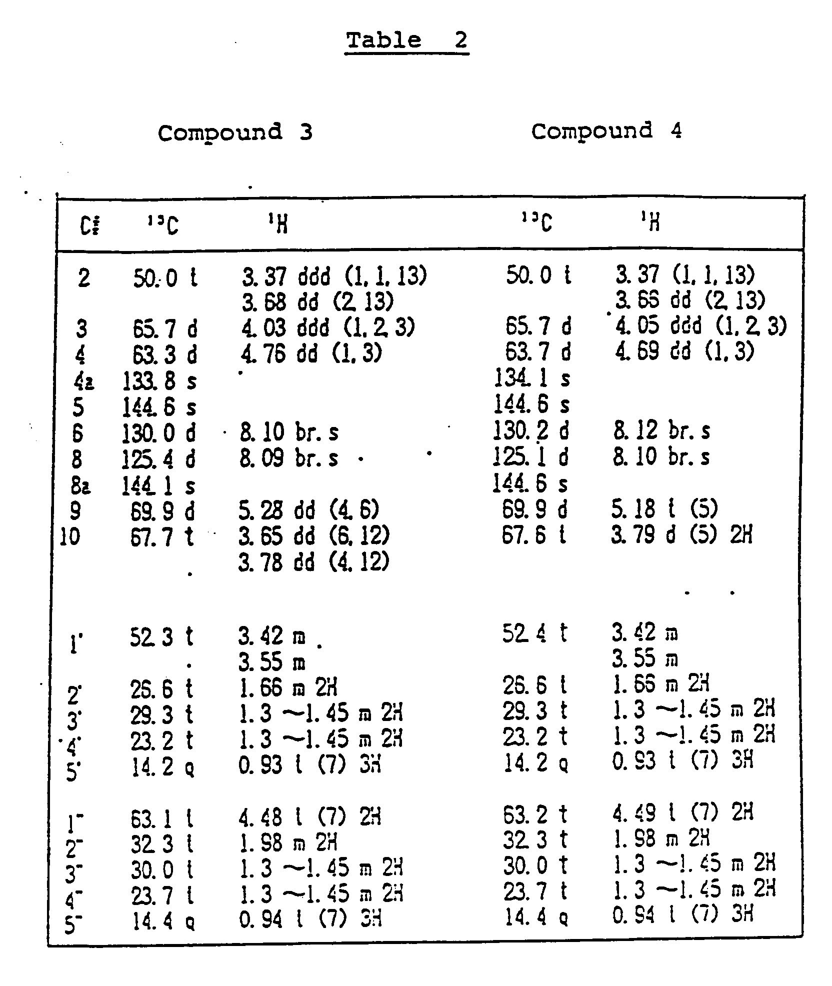

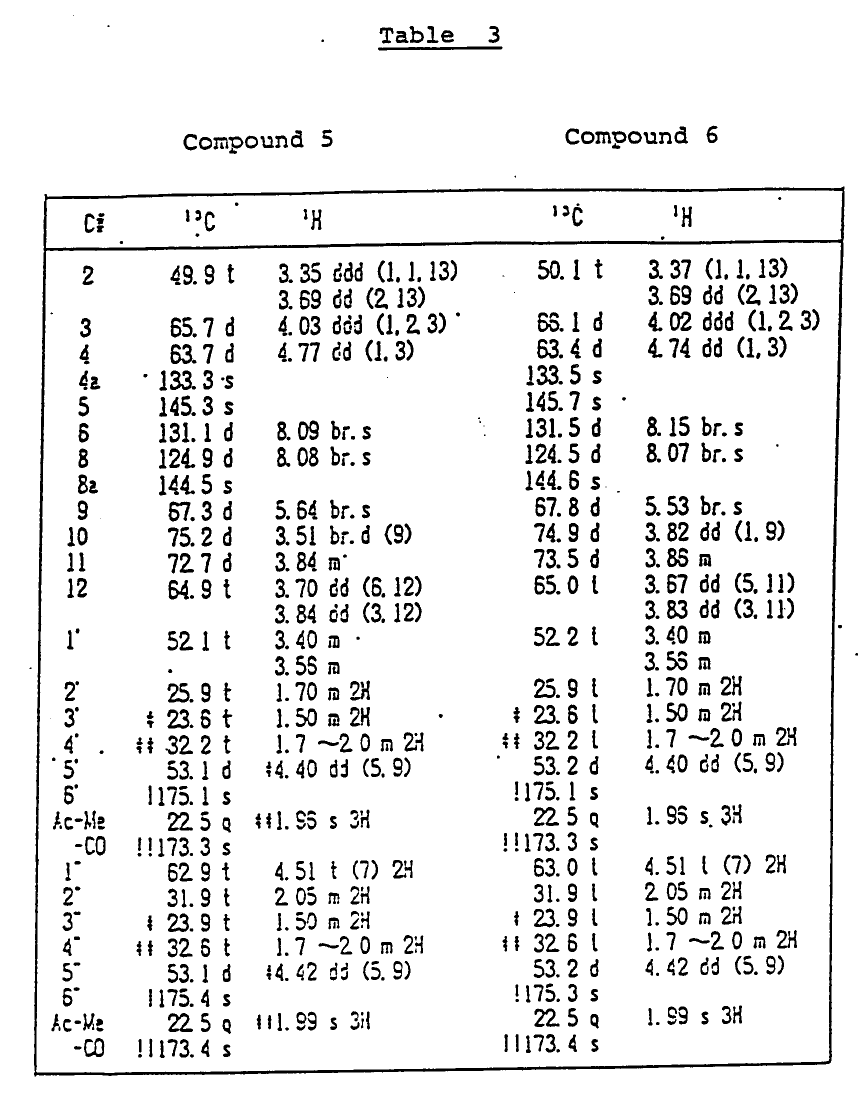

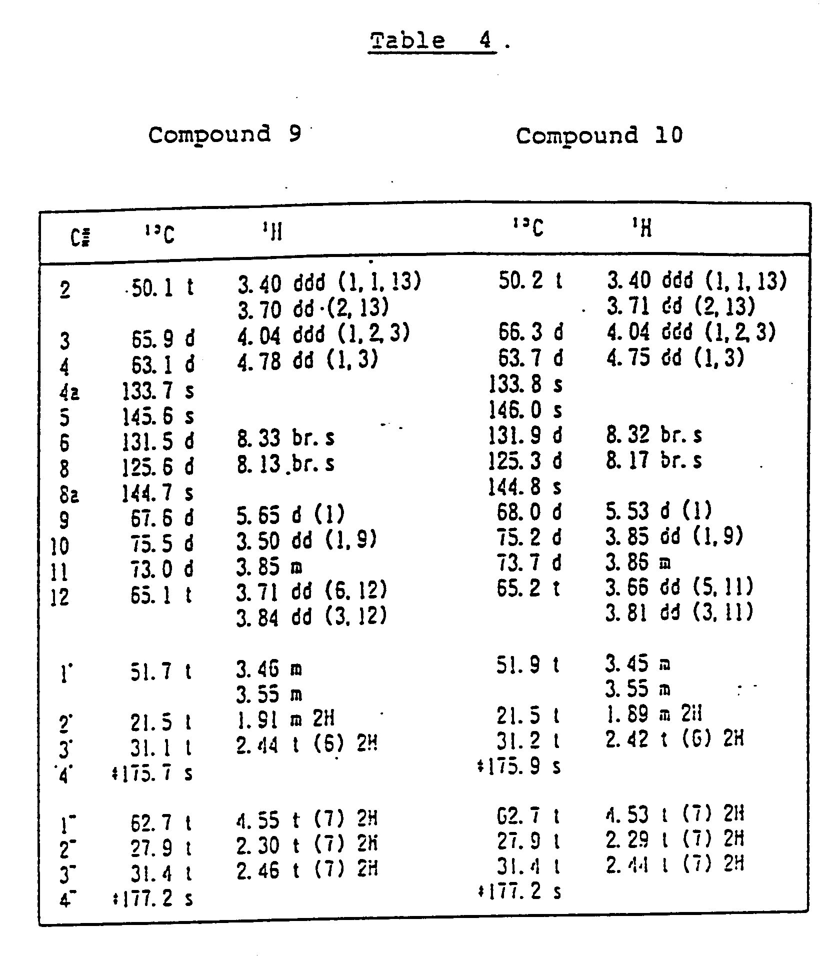

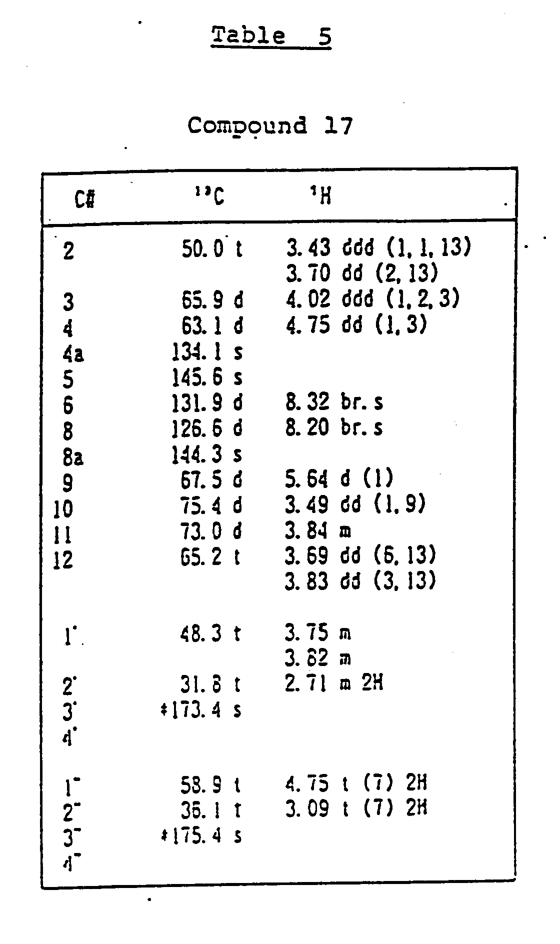

### Example 4

Compounds 5 and 6 of the present invention are combined with a carrier protein using water-soluble carbodiimide in accordance with the usual coupling reaction to prepare antibodies using the compounds 5 and 6 as haptens. The preparation of the antibody was conducted in rabbits by a method which is commonly used. The antibody prepared was capable of recognizing the compound of the present invention in vitro and was also capable of immunochemically discriminating the saccharized protein in vitro and the crosslinked protein in the basement membrane of glomerulus of model rats suffering from diabetic nephrosis induced by streptozotocin.

The compounds 5 and 6 of the present invention are thought to exhibit a high relationship with the second-stage products of the Maillard reaction in terms of chemical structures and hence are practically very highly useful for diagnostic purposes, etc.

## Claims

1. A pyridinium derivative represented by the general formula (I) or (II) 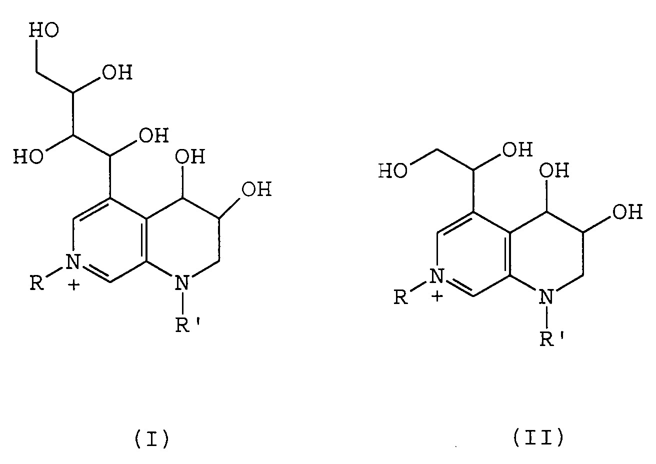 wherein R and R', which may be same or different, are linear, branched or cyclic C₁₋₆ alkyl groups optionally substituted by an amino group, a protected amino group and/or a carboxyl group, or a pharmaceutically acceptable salt thereof.

2. A process for the preparation of a pyridinium derivative represented by the general formula (I) or (II), 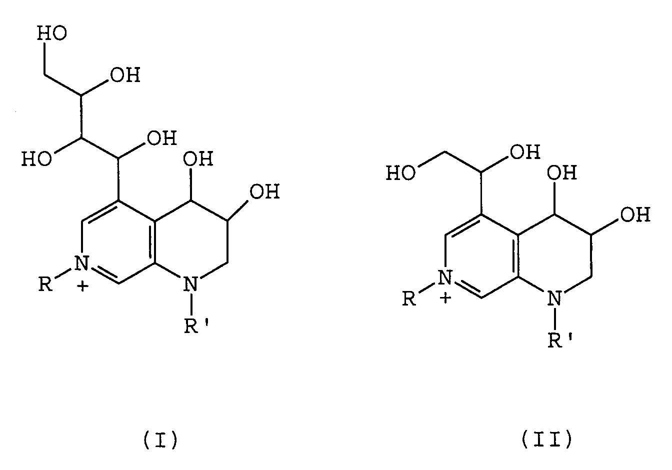 wherein R and R', which may be same or different, are linear, branched or cyclic C₁₋₆ alkyl groups optionally substituted by an amino group, a protected amino group and/or a carboxyl group, or a pharmaceutically acceptable salt thereof, the process comprising the steps of mixing a compound of the formula R-NH₂ or R'-NH₂ (wherein R and R' have the same meanings as defined above) and a hexose or an oligosaccharide consisting of said hexose and allowing the mixture to stand for a suitable time.

3. Use of a pyridinium derivative according to Claim 1 as an indicator for the *in vitro* diagnosis of diabetes, diabetic complications, ageing and diseases accompanied by ageing.

4. Use of a pyridinium derivative according to Claim 1 for the preparation of a diagnostic agent which is suitable for the diagnosis of diabetes, diabetic complications, ageing and diseases accompanied by ageing in men.

5. Use of a pyridinium derivative according to Claim 1 as a hapten for the preparation of an antibody in a manner which is known *per se*.

6. An antibody obtainable by using a pyridinium derivative according to Claim 1 in a process which is known *per se*, the antibody being suitable as an indicator for the diagnosis of diabetes, diabetic complications, ageing and diseases accompanied by ageing and for the evaluation of effects of pharmaceuticals such as therapeutic agents for diabetes, for diabetic complications, preventive agents for ageing and therapeutic agents for diseases accompanied by ageing.

## Patentansprüche

1. Ein Pyridiniumderivat dargestellt durch die allgemeine Formel (I) oder (II) 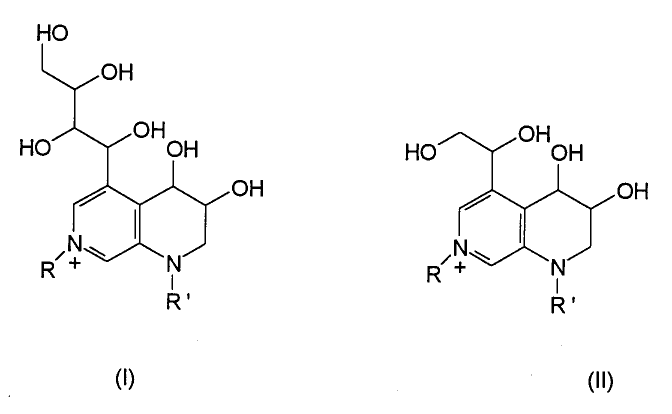 worin R und R', die gleich oder unterschiedlich sein können, lineare, verzweigte oder zyklische C₁₋₆ Alkylgruppen, wahlweise substituiert durch eine Aminogruppe, eine geschützte Aminogruppe und/oder eine Carboxylgruppe, oder ein pharmazeutisch annehmbares Salz davon sind.

2. Ein Verfahren für die Herstellung eines Pyridiniumderivats dargestellt durch die allgemeine Formel (I) oder (II) 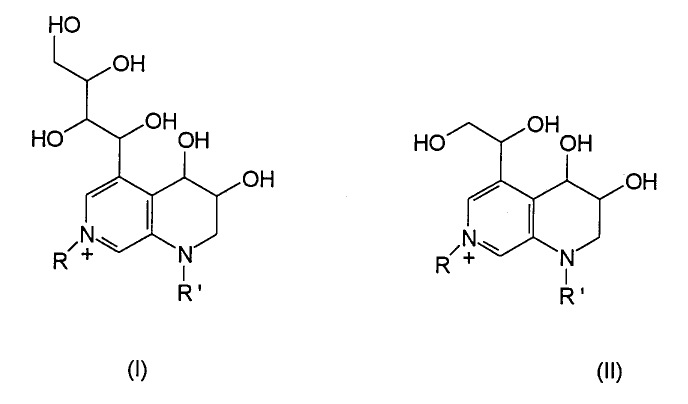 worin R und R', die gleich oder unterschiedlich sein können, lineare, verzweigte oder zyklische C₁₋₆ Alkylgruppen, wahlweise substituiert durch eine Aminogruppe, eine geschützte Aminogruppe und/oder eine Carboxylgruppe, oder ein pharmazeutisch annehmbares Salz davon sind, wobei das Verfahren die Schritte umfaßt: Mischen einer Verbindung der Formel R-NH₂ oder R'-NH₂ (worin R und R' die gleiche Bedeutung wie oben definiert haben) und einer Hexose oder eines Oligosaccharids bestehend aus der Hexose und Stehenlassen der Mischung für eine geeignete Zeit.

3. Verwendung eines Pyridiniumderivats nach Anspruch 1 als ein Indikator für die in vitro Diagnose von Diabetes, diabetischen Komplikationen, Altern und altersbedingten Krankheiten.

4. Verwendung eines Pyridiniumderivats nach Anspruch 1 für die Herstellung eines Diagnostikums, welches geeignet ist für die Diagnose von Diabetes, diabetischen Komplikationen, Altern und altersbedingten Krankheiten von Männern.

5. Verwendung eines Pyridiniumderivats nach Anspruch 1 als ein Hapten für die Herstellung eines Antikörpers in einer Weise die per se bekannt ist.

6. Ein Antikörper erhältlich durch Verwenden eines Pyridiniumderivats nach Anspruch 1 in einem Verfahren, welches per se bekannt ist, wobei der Antikörper geeignet ist als Indikator für die Diagnose von Diabetes, diabetischen Komplikationen, Altern und altersbedingten Krankheiten, und für die Auswertung von Auswirkungen von Pharmazeutika, wie Therapeutika für Diabetes, für diabetische Komplikationen, Präventivwirkstoffe für Altern und Therapeutika für altersbedingte Krankheiten.

## Revendications

1. Dérivé pyridinium représenté par la formule générale (I) ou la formule générale (II): 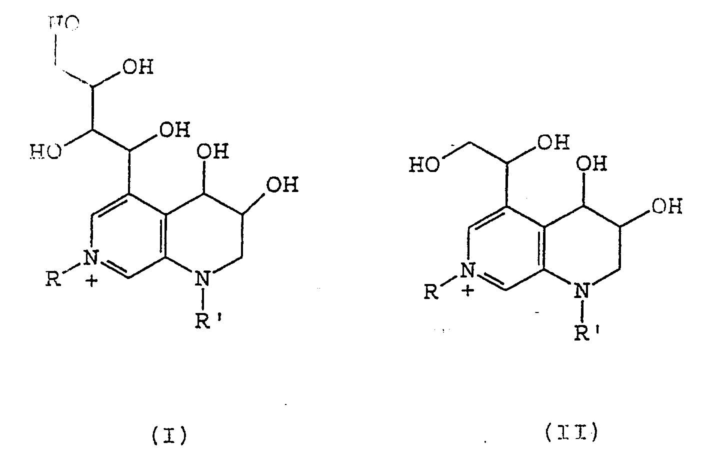 dans lesquelles R et R', qui peuvent être les mêmes ou différents, sont des- groupes alkyle en C₁₋₆ linéaires, ramifiés ou cycliques, le cas échéant substitués avec un groupe amine, un groupe amine protégé et/ou un groupe carboxyle, ou un sel de ce dérivé, acceptable sur le plan pharmaceutique.

2. Procédé pour préparer un dérivé pyridinium représenté par la formule générale (I) ou la formule générale (II): 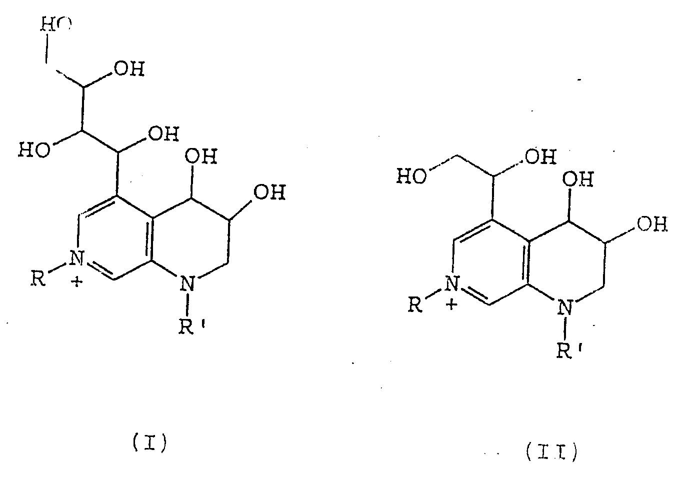 dans lesquelles R et R', qui peuvent être les mêmes ou différents, sont des groupes alkyle en C₁₋₆ linéaires, ramifiés ou cycliques, le cas échéant substitués avec un groupe amine, un groupe amine protégé et/ou un groupe carboxyle, ou un sel de ce dérivé, acceptable sur le plan pharmaceutique, le procédé comprenant les étapes consistant à mélanger un composé de la formule R-NH₂ ou de la formule R'-NH₂ (dans lesquelles R et R' ont la même signification que celle donnée plus haut) et un hexose ou un oligosaccharide contenant ledit hexose, et à laisser le mélange au repos pendant une durée de temps appropriée.

3. Utilisation d'un dérivé pyridinium selon la revendication 1, comme indicateur pour un diagnostic *in vitro* d'un diabète, de complications liées à un diabète, d'un vieillissement et de maladies associées au vieillissement.

4. Utilisation d'un dérivé pyridinium selon la revendication 1, pour la préparation d'un agent diagnostique utile pour le diagnostic d'un diabète, de complications liées à un diabète, d'un vieillissement et de maladies associées au vieillissement chez l'homme.

5. Utilisation du dérivé pyridinium selon la revendication 1, comme haptène pour la préparation d'un anticorps, d'une manière connue en soi.

6. Anticorps obtenu en utilisant un dérivé pyridinium selon la revendication 1, dans un procédé qui est connu en soi, l'anticorps étant utile comme indicateur pour le diagnostic d'un diabète, de complications associées à un diabète, d'un vieillissement et de maladies associées à un vieillissement et comme indicateur pour évaluer les effets de composés pharmaceutiques tels que des agents thérapeutiques agissant contre le diabète et les complications associées à un diabète, des agents de prévention du vieillissement et des agents thérapeutiques agissant contre les maladies associées au vieillissement.
